(19) Europäisches Patentamt
European Patent Office
Office européen des brevets

(11) Veröffentlichungsnummer: **0 293 752**
**A2**

(12) **EUROPÄISCHE PATENTANMELDUNG**

(21) Anmeldenummer: **88108378.6**

(22) Anmeldetag: **26.05.88**

(51) Int. Cl.[4]: **C07C 57/03 , C07C 51/08 , C07C 51/377 , C07C 51/41 , C07C 121/36**

(30) Priorität: **02.06.87 DE 3718803**

(43) Veröffentlichungstag der Anmeldung: **07.12.88 Patentblatt 88/49**

(84) Benannte Vertragsstaaten: **DE FR GB NL**

(71) Anmelder: **Desitin Arzneimittel GmbH**
**Weg beim Jäger 214**
**D-2000 Hamburg 63(DE)**

(72) Erfinder: **Schäfer, Helmut**
**Stegener Weg 40**
**D-2061 Kayhude(DE)**

(74) Vertreter: **UEXKÜLL & STOLBERG**
**Patentanwälte**
**Beselerstrasse 4**
**D-2000 Hamburg 52(DE)**

(54) **Verfahren zur Herstellung von E-2-Propyl-2-pentensäure und physiologisch verträglichen Salzen derselben.**

(57) Es wird ein neues Verfahren zur Herstellung von E-2-Propyl-2-pentensäure sowie von deren physiologisch verträglichen Salzen beschrieben, bei dem Di-n-propylketoncyanhydrin als Ausgangsverbindung verwendet wird. Die Verbindung wird entweder

a) mit Thionylchlorid dehydratisiert und das gebildete Säurenitril anschließend mit einem stöchiometrischen Überschuß von Kaliumhydroxid in Glycerin verseift oder

b) zunächst in die 2-Hydroxy-2-propylpentansäure überführt und diese anschließend in Gegenwart einer weniger als stöchiometrischen Menge eines tertiären Amins bei einer Temperatur von mindestens 200° C dehydratisiert.

Die freie Säure wird gegebenenfalls,vorzugsweise unter Verwendung der entsprechenden Salze der Kohlensäure in wäßriger Acetonlösung,in die Salzform überführt.

EP 0 293 752 A2

## Verfahren zur Herstellung von E-2-Propyl-2-pentensäure und physiologisch verträglichen Salzen derselben

2-Propyl-2-pentensäure ein Derivat der Valproinsäure, hat in neuerer Zeit großes Interesse als mögliches Therapeutikum zur Behandlung der Epilepsie hervorgerufen. Während nämlich unter einer konventionellen Behandlung mit Valproinsäure in Einzelfällen heptotoxische und teratogene Eigenschaften zu befürchten sind, zeigen vergleichende Tierversuche mit E-2-Propyl-2-pentensäure, daß diese bei mindestens gleichwertiger therapeutischer Wirksamkeit keine lebertoxischen oder teratogenen Effekte hervorbringt (vgl. W. Löscher, Drugs of the Future, 10, 389-391 (1985)). Die toxikologisch günstigen Eigenschaften wurden speziell bei dem E-Isomeren der 2-Propyl-2-pentensäure beobachtet.

Die E-2-Propyl-2-pentensäure ist eine kristalline Verbindung, deren Schmelzpunkt bei 38 bis 40 °C liegt und die als solche bereits seit langem bekannt ist.

Die Herstellung von 2-Propyl-2-pentensäure aus dem Cyanhydrin des Heptanon-4 der Formel II

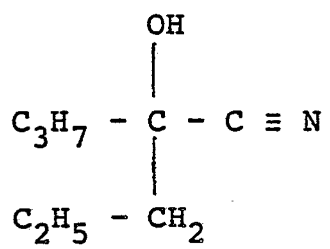

II

ist bereits bekannt.

Durch Umsetzen der Verbindung gemäß Formel II mit konzentrierter Schwefelsäure kann zunächst das entsprechende Säureamid und anschließend die Säure hergestellt werden. Bei diesem Verfahren entsteht jedoch neben weiteren Zersetzungs- und Umlagerungsprodukten überwiegend das Z-Isomere der gewünschten Säure.

Es ist demgemäß Aufgabe der Erfindung, ein Verfahren zu schaffen, bei dem die Bildung des E-Isomeren der 2-Propyl-2-pentensäure begünstigt ist.

Zur Lösung der Aufgabe wird das Verfahren gemäß Anspruch 1 vorgeschlagen. Besondere Ausbildungsformen ergeben sich aus den Unteransprüchen.

Erfindungsgemäß hat es sich überraschenderweise gezeigt, daß man ausgehend von der Formel II bevorzugt das E-Isomere der 2-Propyl-2-pentensäure erhält, wenn man entweder

a) die Ausgangsverbindung mit Thionylchlorid dehydratisiert und anschließend das vorwiegend als Z-Isomeres gebildete Säurenitril mit einem stöchiometrischen Überschuß von Kaliumhydroxid in Glycerin bei Temperaturen von mindestens 130 °C verseift oder

b) die Ausgangsverbindung zunächst in bekannter Weise in die 2-Hydroxy-2-propylpentansäure überführt und diese anschließend in Gegenwart einer substöchiometrischen Menge eines tertiären Amins, vorzugsweise N-Methylpyrrolidin, bei einer Temperatur von mindestens 200 °C dehydratisiert.

Auf die Möglichkeit der Verseifung von Nitrilen mit Hilfe verdünnten KOH-Lösungen in Glycerin bei Temperaturen oberhalb von 150 °C ist in der Literatur schon hingewiesen worden (vgl. Houben-Weyl). Es war jedoch nicht zu erwarten, daß bei den erfindungsgemäß verwendeten hohen Konzentrationen von KOH in Glycerin und unter mehrstündigem Kochen ein Rohprodukt erhalten werden würde, welches zu mehr als 60% aus dem E-Isomeren der gewünschten Säure besteht. Die Umsetzung der Ausgangsverbindung gemäß Formel II mit Thionylchlorid liefert nämlich zunächst überwiegend das Z-Isomere der Verbindung gemäß Formel III. Diese Umsetzung wird bei 70 bis 100 und vorzugsweise bei 80 bis 90 °C 2 bis 4 Stunden lang am Rückfluß durchgeführt. Vorzugsweise wird das Thionylchlorid in einer solchen Menge verwendet, daß es zum Lösen der Ausgangsverbindung und zum Erreichen der genannten Rückflußtemperatur ausreicht.

Das Reaktionsgemisch wird anschließend mit Wasser versetzt, um überschüssiges Thionylchlorid zu beseitigen. Die organische Phase wird dann unter Verwendung von beispielsweise Methyl-tert.-butylether abgetrennt. Die vereinigten organischen Phasen werden anschließend gewaschen und destilliert, um 2-Propyl-2-pentennitril überwiegend als Z-Isomeres zu gewinnnen. In dem darauffolgenden Verfahrensschritt wird zunächst durch langsames Erhitzen eine Lösung von KOH in Glycerin hergestellt und das Säurenitril anschließend zugetropft. Die erfindungsgemäß verwendete KOH-Menge liegt oberhalb der stöchiometrischen Menge und das Äquivalenzverhältnis von Kaliumhydroxid zu Säurenitril in der Mischung beträgt vorzugsweise 3 : 1 bis 4,5 : 1.

Das Reaktionsgemisch wird anschließend 6 bis 10, vorzugsweise 8 Stunden lang am Rückfluß gekocht, wobei eine Temperatur von mindestens 130 °C, vorzugsweise jedoch 140 bis 150 °C erreicht wird. Nach Abschluß der Reaktionszeit wird das Gemisch beispielsweise mit konzentrierter Salzsäure angesäuert und die sich abscheidende Ölphase in einem organischen Lösungsmittel wie beispielsweise n-Hexan aufgenommen. Nach Waschen der organischen Phase erhält man durch Eindampfen ein Rohprodukt, welches bevorzugt das E-Isomere der 2-Propyl-2-pentensäure enthält. Diese kann durch Feindestillation aus dem Rohprodukt isoliert werden.

Wie bereits oben erläutert, kann die Ausgangsverbindung gemäß Formel II ferner zunächst in die 2-Hydroxy-2-propylpentansäure überführt und anschließend dehydratisiert werden, um das erfindungsgemäß gewünschte Endprodukt zu gewinnen.

Auch die thermische Wasserabspaltung aus 2-Hydroxy-2-propylpentansäure ist bereits bekannt. Nach dem bekannten Verfahren besteht das erhaltene Reaktionsprodukt jedoch zum überwiegenden Anteil aus dem Z-Isomeren. Erfindungsgemäß hat es sich überraschenderweise gezeigt, daß durch Zusatz von weniger als der stöchiometrischen Menge eines tertiären Amins die thermische Wasserabspaltung aus der 2-Hydroxy-2-propylpentansäure bevorzugt zur Bildung des E-Isomeren der en-Säure führt.

Die 2-Hydroxy-2-propylpentansäure kann aus der Ausgangsverbindung der Formel II beispielsweise durch Verseifen mit konzentrierter Salzsäure gewonnen werden. Die Säure wird anschließend mit einem tertiären Amin, vorzugsweise N-Methylpyrrolidin auf eine Temperatur oberhalb 200 °C erhitzt. Die Menge des verwendeten Amins beträgt vorzugsweise 5 bis 10 Mol% bezogen auf die Ausgangsverbindung der Formel II und die Reaktion wird etwa 1 Stunde lang bei einer Temperatur von 200 bis 250, vorzugsweise von 230 bis 240 °C durchgeführt. Sie ist nach etwa 1 Stunde beendet. Der ölige Rückstand wird auch in diesem Fall nach Ansäuern in einem organischen Lösungsmittel wie beispielsweise n-Hexan aufgenommen, mit verdünnter Mineralsäure gewaschen und anschließend destilliert.

Die freie Säure kann, falls gewünscht, in üblicher Weise in die Form eines physiologisch verträglichen Salzes überführt werden.

Gemäß einer besonders bevorzugten Ausführungsform wird die Umsetzung in wäßrigem Aceton als Lösungsmittel in Gegenwart des entsprechenden Salzes der Kohlensäure durchgeführt. Soll beispielsweise das Natriumsalz der E-2-Propyl-2-pentensäure hergestellt werden, so wird vorzugsweise Natriumcarbonat in einer Lösung der freien Säure in einem Aceton/Wassergemisch einige Stunden lang am Rückfluß gekocht. Nach Abkühlen auf eine Temperatur unterhalb 0 °C wird dann das Natriumsalz als Niederschlag feiner weißer Kristalle erhalten.

Die Erfindung wird nachfolgend anhand von Beispielen erläutert.

## Beispiel 1

### Herstellung von 2-Propyl-2-pentennitril

In einem 2 l-Kolben wurden 535 g Thionylchlorid vorgelegt. Bei 30 bis 35 °C Innentemperatur wurden 353 g Di-n-propylketoncyanhydrin im Laufe von 3 Stunden zugetropft. Anschließend wurde das Reaktionsgemisch 3 Stunden lang unter leichtem Rückfluß auf 80 bis 90 °C erhitzt, wobei die über den Rückflußkühler entweichenden Gase HCl und $SO_2$ von einer 40%igen Natronlauge absorbiert wurden. Das Reaktionsgemisch wurde danach mit 390 ml Wasser zur Zersetzung überschüssigen Thionylchlorids verrührt. Nach Abtrennung der organischen Phasen wurde die wäßrige Phase mit 200 ml Methyl-tert.-butylether gewaschen. Die vereinigten organischen Phasen wurden nach Waschen mit 10%iger Natronlauge und Wasser über eine Kolonne destilliert.

Die Ausbeute betrug 254 g entsprechend 82,5% der Theorie, $Kp_{17}$: 62 - 64 °C.

Das auf diese Weise erhaltene 2-Propyl-2-pentennitril war ein Gemisch der Z- und E-Isomeren im Verhältnis von etwa 80 : 20.

## Beispiel 2

Herstellung von E-2-Propyl-2-pentensäure aus 2-Propyl-2-pentennitril (Gemisch Z/E etwa 80 : 20)

In einem 4 l-Kolben wurden 1425 g Glycerin vorgelegt. Es wurden 470 g KOH zugegeben und die Mischung langsam erhitzt, wobei sich das Kaliumhydroxid bei etwa 100° C löste. Nach Erreichen einer Temperatur von 140° C wurden im Verlaufe von 1 1/2 Studen 264,4 g 2-Propyl-2-pentennitril zugetropft, wobei die Temperatur auf 140 bis 145° C gehalten wurde. Zu Beginn trat ein leichtes Schäumen ein. Das Reaktionsgemisch wurde 8 Stunden lang am Rückfluß gekocht, wobei Ammoniak entwich, und danach mit 1700 ml Wasser versetzt. Nach dem Abkühlen hatte sich auf der klaren gelben Lösung eine etwa 2 cm dicke flockige Schaumschicht abgesetzt. Das Reaktionsgemisch wurde mit 620 ml konzentrierte Salzsäure auf pH 1 angesäuert. Danach wurde das ausgeschiedene Öl mit 100 ml n-Hexan aufgenommen und die wäßrige Phase noch zweimal mit je 400 ml n-Hexan extrahiert. Nach Waschen der organischen Phase mit Wasser und Trocknen wurde durch Eindampfen bei 80 mbar und 40° C ein Rückstand von 260 g erhalten. Durch Feindestillation an einer wirksamen Kolonne erhielt man daraus 145,4 g E-2-Propyl-2-pentensäure entsprechend 50,4% der Theorie. Der $Kp_{15}$ betrug 135 bis 136° C Das Destillat erstarrte zu einer kristallinen Masse mit dem Schmelzpunkt 38° C

## Beispiel 3

Herstellung von E-2-Propyl-2-pentensäure aus 2-Hydroxy-2-propylpentansäure

10 g 2-Hydroxy-2-propylpentansäure, auf übliche Weise durch Verseifen des Di-n-propylketoncyanhydrins mit konzentrierter Salzsäure gewonnen, und 1 g N-Methylpyrrolidin wurden im Ölbad auf über 200° C erhitzt. Bei einer Innentemperatur von 230° C begann die Abspaltung von Wasser, das über eine aufgesetzte Kolonne aus dem Reaktionsgemisch entfernt wurde. Die Reaktion bei 230 bis 240° C war nach etwa 1 Stunde beendet. Der Rückstand im Reaktionskolben wurde n-Hexan aufgenommen, mit verdünnter Salzsäure gewaschen und destilliert. Durch Feindestillation über eine wirksame Kolonne erhielt man 4,5 g E-2-Propyl-2-pentensäure entsprechend 50,7% der Theorie. Der $Kp_{15}$ betrug 135 bis 136° C.

## Beispiel 4

Herstellung des Natriumsalzes der E-2-Propyl-2-pentensäure

10 g feingemahlenes wasserfreies Natriumcarbonat wurden in einer Lösung von 142,2 g E-2-Propyl-2-pentensäure in 1600 ml Aceton und 54 ml Wasser 6 Stunden lang unter Rückfluß gekocht und die Lösung anschließend filtriert. Aus dem Filtrat kristallisierte nach Abkühlen auf etwa -5° C das Natriumsalz in feinen, weißen Kristallen aus. Die Ausbeute nach Aufarbeitung der Mutterlauge war nahezu quantitativ. Der Schmelzpunkt der gebildeten Kristalle lag über 300° C. Das erhaltene Salz war leicht wasserlöslich.

**Ansprüche**

1. Verfahren zur Herstellung von E-2-Propyl-2-pentensäure der Formel I

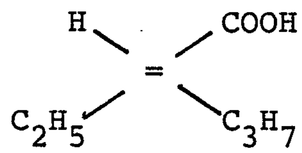

I

oder physiologisch verträglichen Salzen derselben unter Verwendung von Di-n-propylketoncyanhydrin der Formel II

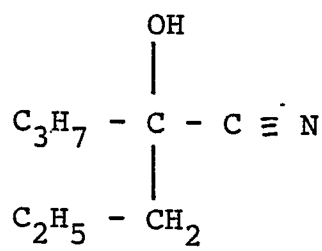

II

als Ansgangssubstanz, **dadurch gekennzeichnet,**
a) i) daß man die Verbindung der Formel II bei 70 bis 100 °C mit Thionylchlorid umsetzt,
ii) das in Stufe i) überwiegend als Z-Isomeres erhaltene 2-Propyl-2-pentennitril der Formel III

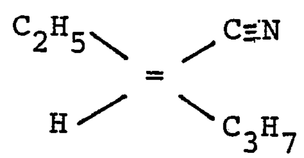

III

bei einer Temperatur von mindestens 130 °C mit einem stöchiometrischen Überschuß von KOH in Glycerin als Lösungsmittel umsetzt,
iii) die in Stufe ii) erhaltene E-2-Propyl-2-pentensäure in üblicher Weise aus der Mischung abtrennt und
iv) gegebenenfalls die in Stufe iii) erhaltene freie Säure in die Form eines physiologisch verträglichen Salzes derselben überführt, oder
b) i) die Verbindung der Formel II auf bekannte Weise durch Verseifen in 2-Hydroxy-2-propylpentansäure der Formel IV

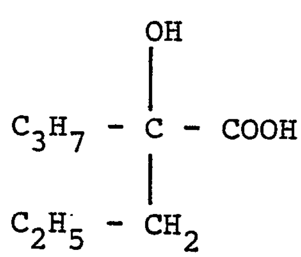

IV

überführt,
ii) die Verbindung der Formel IV bei einer Temperatur von mindestens 200 °C mit einer weniger als stöchiometrischen Menge eines tertiären Amins umsetzt,
iii) die in Stufe ii) gebildete E-2-Propyl-2-pentensäure in üblicher Weise aus der Mischung abtrennt und
iv) die in Stufe iii) erhaltene freie Säure gegebenenfalls in die Form eines physiologisch verträglichen Salzes derselben überführt.

2. Verfahren nach Anspruch 1, **dadurch gekennzeichnet,** daß man die Umsetzung gemäß Stufe ai) am Rückfluß in einer zum Lösen und zum Erreichen einer Rückflußtemperatur von 70 bis 100, vorzugsweise 80 bis 90 °C ausreichenden Menge Thionylchlorid durchführt.

3. Verfahren nach einem der Ansprüche 1 oder 2, **dadurch gekennzeichnet,** daß das Äquivalenzverhältnis von Kaliumhydroxid zu Säurenitril in Stufe aii) 3 : 1 bis 4,5 : 1 beträgt.

4. Verfahren nach den Ansprüchen 1 bis 3, **dadurch gekennzeichnet,** daß man die Umsetzung in Stufe aii) bei 130 bis 160, vorzugsweise 140 °C bis 150 °C durchführt.

5. Verfahren nach den Ansprüchen 1 bis 4, **dadurch gekennzeichnet,** daß man in Stufe aii) wasserfreies Glycerin verwendet.

6. Verfahren nach Anspruch 1, **dadurch gekennzeichnet,** daß man in Stufe bii) als tertiäres Amin N-Methylpyrrolidin verwendet.

7. Verfahren nach den Ansprüchen 1 oder 5, **dadurch gekennzeichnet,** daß man das tertiäre Amin in einer Menge von 5 bis 10 Mol% bezogen auf die Ausgangsverbindung der Formel II verwendet.

8. Verfahren nach einem der Ansprüche 1, 5 oder 6, **dadurch gekennzeichnet,** daß die Temperatur in Stufe bii) 200 bis 250, vorzugsweise 230 bis 240 °C beträgt.

9. Verfahren nach Anspruch 1, **dadurch gekennzeichnet,** daß man zur Herstellung eines physiologisch verträglichen Salzes der E-2-Propyl-2-pentensäure die freie Säure mit dem korrespondierenden Salz der Kohlensäure in einer Mischung aus Aceton und Wasser als Lösungsmittel umsetzt.

10. Verfahren nach Anspruch 9, **dadurch gekennzeichnet,** daß man als Salz der Kohlensäure Natriumcarbonat verwendet.